# FASCICULE DE BREVET EUROPEEN

(11) **EP 1 093 807 B1**
(45) Date de publication et mention de la délivrance du brevet: **04.04.2007**
(21) Numéro de dépôt: 00402738.9
(22) Date de dépôt: 05.10.2000
(51) Int. Cl.: A61Q 5/12, A61K 8/891, A61K 8/73

(54) **Compositions cosmétiques contenant un copolymère vinyldiméthicone/diméthicone en émulsion aqueuse et un épaississant et leurs utilisations**
Kosmetische wässerige Emulsionzusammensetzung enthaltend ein Vinyldimethicon/dimethicon Copolymer und ein Verdickungsmittel, und ihre Anwendungen
Cosmetic aqueous emulsion composition containing a vinyldimethicone/dimethicone copolymer and a thickener, and their uses

(30) Priorité: 20.10.1999 FR 9913099
(43) Date de publication de la demande: 25.04.2001
(73) Titulaire: L'ORÉAL, 75008 Paris (FR)
(72) Inventeur: Decoster, Sandrine, 95210 Saint Gratien (FR); Douin, Véronique, 75017 Paris (FR); Bailly, Virginie, 92110 Clichy (FR)
(74) Mandataire: Le Blainvaux Bellegarde, Françoise

(56) Documents cités:
- EP-A- 0 787 758
- EP-A- 0 874 017
- FR-A- 2 739 284
- FR-A- 2 742 657
- FR-A- 2 748 392
- FR-A- 2 755 854
- FR-A- 2 770 523

## Description

La présente invention concerne de nouvelles compositions cosmétiques comprenant dans un milieu cosmétiquement acceptable au moins un copolymère diméthicone à insaturation éthylénique/diméthicone tel que défini dans la revendication 1 de viscosité comprise entre 10⁶ et 100.10⁶ cP en émulsion aqueuse et au moins un épaississant non cellulosique choisi parmi les gommes de xanthane, de scléroglucane, de gellane, de rhamsan, les alginates, la maltodextrine, l'amidon et ses dérivés, la gomme de karaya, la farine de caroube, les gommes de guar, les polyéthylèneglycols (PEG) et leurs dérivés, les polyacrylamides.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe), un toucher désagréable, chargé, un raidissement des cheveux, et une adhésion interfibres affectant le coiffage, en particulier lors d'utilisations répétées. Ces inconvénients sont accentués dans le cas de cheveux fins, qui manquent de nervosité et de volume.
En résumé, il s'avère que les compositions cosmétiques actuelles contenant des silicones, ne donnent pas complètement satisfaction.

La demanderesse a maintenant découvert que l'association d'au moins un copolymère siliconé particulier tel que défini dans la revendication 1 de viscosité comprise entre 10⁶ et 100.10⁶ cP en émulsion aqueuse avec des épaississants non cellulosique choisis parmi les gommes de xanthane, de scléroglucane, de gellane, de rhamsan, les alginates, la maltodextrine, l'amidon et ses dérivés, la gomme de karaya, la farine de caroube, les gommes de guar, les polyéthylèneglycols (PEG) et leurs dérivés, les polyacrylamides, permet de remédier à ces inconvénients.

Ainsi, à la suite d'importantes recherches menées sur la question, il a maintenant été trouvé par la Demanderesse qu'en utilisant l'association d'au moins un copolymère siliconé tel que défini dans la revendication 1 particulier de viscosité comprise entre 10⁶ et 100.10⁶ cP en émulsion aqueuse avec des épaississants non cellulosiques choisis parmi les gommes de xanthane, de scléroglucane, de gellane, de rhamsan, les alginates, la maltodextrine, l'amidon et ses dérivés, la gomme de karaya, la farine de caroube, les gommes de guar, les polyéthylèneglycols (PEG) et leurs dérivés, les polyacrylamides, dans une composition en particulier pour les cheveux, il est possible de limiter, voire supprimer, les problèmes généralement liés à l'emploi de telles compositions, à savoir en particulier l'alourdissement (toucher chargé lors d'applications répétées), le manque de lissage et de douceur des cheveux, tout en conservant les autres propriétés cosmétiques avantageuses qui sont attachés aux compositions à base d'agents conditionneurs.
Cette association apporte des propriétés cosmétiques (lissage, légèreté, douceur) sans phénomène de regraissage des fibres kératiniques.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins une émulsion aqueuse d'au moins un copolymère siliconé défini ci-dessous, ledit copolymère ayant une viscosité comprise entre 10⁶ et 100.10⁶ cP et au moins un agent épaississant non cellulosique choisi parmi les gommes de xanthane, de scléroglucane, de gellane, de rhamsan, les alginates, la maltodextrine, l'amidon et ses dérivés, la gomme de karaya, la farine de caroube, les gommes de guar, les polyéthylèneglycols (PEG) et leurs dérivés, les polyacrylamides.

Un autre objet de l'invention concerne l'utilisation d'une émulsion aqueuse d'au moins un copolymère siliconé défini ci-dessous de viscosité comprise entre 10⁶ et 100.10⁶ cP dans, ou pour la fabrication d'une composition cosmétique comprenant un agent épaississant non cellulosique choisi parmi les gommes de xanthane, de scléroglucane, de gellane, de rhamsan, les alginates, la maltodextrine, l'amidon et ses dérivés, la gomme de karaya, la farine de caroube, les gommes de guar, les polyéthylèneglycols (PEG) et leurs dérivés, les polyacrylamides.

Les différents objets de l'invention vont maintenant être détaillés. L'ensemble des significations et définitions des composés utilisés dans la présente invention données ci-dessous sont valables pour l'ensemble des objets de l'invention.

Le copolymère siliconé a généralement une viscosité dynamique, mesurée à la température d'environ 25°C et au taux de cisaillement de 0,01 Hz pour une contrainte de 1500 Pa, comprise entre 10⁶ et 100.10⁶ cP et de préférence comprise entre 5.10⁶ cP et 30,10⁶ cP.

Toutes les mesures de viscosités dynamiques données dans la présente demande ont été effectuées à une température d'environ 25°C, sur un Carri-Med CSL2-500.

Le copolymère siliconé présent dans la composition selon l'invention se trouve sous la forme d'émulsion aqueuse.
Par émulsion aqueuse, on entend une émulsion de type huile-dans-eau dans laquelle le copolymère siliconé est dispersé sous forme de particules ou de gouttelettes dans la phase aqueuse formant la phase continue de l'émulsion.
Cette émulsion peut être stabilisée par un système émulsionnant usuel.
Cette émulsion de silicone peut avoir une taille de gouttelettes ou de particules de silicone allant de 10 nm à 50 µm, et de préférence de 0,3 µm à 20 µm.
La taille des particules est mesurée par granulométrie laser.
Le système émulsionnant comprend des tensioactifs employés habituellement dans les émulsions de silicone. Ces tensioactifs peuvent être non-ioniques, cationiques, anioniques ou amphotères ou leurs mélanges tels que ceux décrits ci-dessous.

Le système émulsionnant représente de 0,5% à 10 % en poids par rapport au poids total de l'émulsion.

Le copolymère siliconé résulte de la réaction d'addition, en présence d'un catalyseur, d'au moins :
- (a) un polysiloxane de formule (I) : 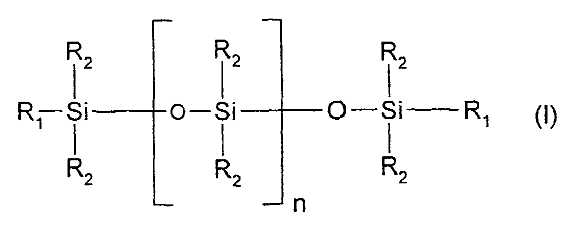 dans laquelle :
   R1 désigne un groupement réagissant par réaction d'addition de chaîne tel que par exemple un atome d'hydrogène, un groupement aliphatique ayant une insaturation éthylénique notamment vinyl, allyl ou héxényl,
   Les groupements R2 de la formule (I) peuvent représenter notamment des groupements alkyle, alcényle, cycloalkyle, aryle, alkylaryle ou hydroxyle, et peuvent comporter en outre, des groupements fonctionnels tels qu'éthers, amines, carboxyles, hydroxyles, thiols, esters, sulfonates, sulfates.
   Les groupements alkyle ont par exemple 1 à 20 atomes de carbone ; les groupements cycloalkyle ont par exemple 5 ou 6 atomes de carbone ; les groupements aryle sont notamment des groupements phényle ; les groupements alcényle ont notamment de 2 à 10 atomes de carbone ; les groupements alkylaryle peuvent avoir de 7 à 20 atomes de carbone.
   Plus particulièrement R2 désigne méthyle.
   n est un entier tel que le polysiloxane de formule (I) ait de préférence une viscosité cinématique comprise entre 1 et 1.10⁶ mm²/s..n varie notamment de 5 à 5000.
- (b) et d'au moins un composé siliconé comprenant au moins un et au plus deux groupements réagissant avec les groupements R1 du polysiloxane (a), l'un au moins des radicaux R1 des composés de type (a) ou (b) désigne un groupe aliphatique ayant une insaturation éthylénique.

Les composés de type (b) sont un autre polysiloxane de type (a) dans lequel les groupements R1 du polysiloxane (b) réagissent avec les groupement R1 du polysiloxane (a).

De préférence, les copolymères siliconés sont notamment obtenus par réaction d'addition, en présence d'un catalyseur d'hydrosilylation (par exemple un catalyseur au platine), d'au moins :
- (a) un alpha,oméga-di vinyl polydiméthylsiloxane, et
- (b) d'un alpha,oméga-di hydrogéno polydiméthylsiloxane.

La viscosité cinématique est par exemple mesurée à 25°C selon la norme ASTM 445 Appendice C.

Les copolymères siliconés selon l'invention sont essentiellement non réticulés.

La synthèse de ces émulsions de silicones est notamment décrite dans la demande EP-A-874017.

De telles émulsions sont notamment commercialisées sous la dénomination DC2-1997 cationic emulsion par la société DOW CORNING. Cette émulsion comprend un copolymère α,ω̅-divinyl diméthicone/α,ω̅-dihydrogénodiméthicone ayant une viscosité dynamique d'environ 15.10⁶ cP, un émulsionnant de type cationique tel que le chlorure de cétyltriméthylammonium, un stabilisant de type hydroxyéthylcellulose et de l'eau.

Le copolymère siliconé est utilisé de préférence en une quantité comprise entre 0,05 et 10% en poids par rapport au poids total de la composition. Plus préférentiellement, cette quantité est comprise entre 0,1 et 5% en poids par rapport au poids total de la composition.

L'émulsion aqueuse du copolymère siliconé représente de 0,5 à 15% en poids du poids total de la composition.

Les épaississants non cellulosiques selon l'invention peuvent être d'origine naturelle ou synthétique.

On peut citer comme épaississants naturels convenant pour l'invention les gommes de xanthane, de scléroglucane, de gellane, de rhamsan, les alginates, la maltodextrine, l'amidon et ses dérivés, la gomme de karaya, la farine de caroube, les gommes de guar. On peut également utiliser ces composés après modification chimique comme par exemple les hydroxypropylguars.

Les agents épaississants purement synthétiques selon l'invention sont avantageusement des polyéthylèneglycols (PEG) et leurs dérivés.

On peut également utiliser avantageusement, en tant qu'agent épaissisant, des polyacrylamides épaississants. Ceux-ci peuvent plus particulièrement être choisis parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide et d'acide 2-acrylamido 2-méthyl propanesulfonique partiellement ou totalement neutralisés éventuellement réticulés.

Comme copolymères réticulés d'acrylamide / acrylate d'ammonium, utilisés conformément à la présente invention, on peut plus particulièrement citer les copolymères acrylamide / acrylate d'ammonium (5/95 en poids) réticulé par un agent de réticulation à polyinsaturation oléfinique, tel que le divinylbenzène, le tétraallyloxyéthane, le méthylène bis-acrylamide, l'éther diallylique, des éthers polyallylpolyglycéryliques ou les éthers allyliques d'alcool de la série des sucres, tels que l'érythritol, le pentaérythritol, l'arabitol, le mannitol, le sorbitol ou le glucose.

Des copolymères analogues sont décrits et préparés dans le brevet français FR-2.416.723 et les brevets US-2.798.053 et US-2.923.692.

On utilise en particulier ce type de copolymère réticulé sous forme d'émulsion eau-dans-huile constituée d'environ 30 % en poids dudit copolymère, 25 % en poids d'huile de paraffine, 4 % en poids de mélange de stéarate de sorbitan et d'un dérivé éthoxylé hydrophile et 41 % en poids d'eau. Une telle émulsion est commercialisée sous la dénomination "BOZEPOL C" par la Société HOECHST.

Les copolymères d'acrylamide et de l'acide 2-acrylamido 2-méthyl propane sulfonique, utilisés conformément à la présente invention, sont des copolymères réticulés par un composé à polyinsaturation oléfinique, tels que ceux évoqués précédemment, et partiellement ou totalement neutralisés par un agent de neutralisation tel que la soude, la potasse, l'ammoniaque ou une amine telle que la triéthanolamine ou la monoéthanolamine.

Ils peuvent être préparés en copolymérisant l'acrylamide et le 2-acrylamido 2-méthylpropane sulfonate de sodium par voie radicalaire au moyen d'agents initiateurs du type azobisisobutyronitrile et par précipitation dans un alcool tel que le tertiobutanol.

On utilise plus particulièrement des copolymères obtenus par copolymérisation de 70 à 55 % en moles d'acrylamide et de 30 à 45 % en moles de 2-acrylamido 2-méthylpropane sulfonate de sodium. L'agent de réticulation étant utilisé à des concentrations de 10⁻⁴ à 4,10⁻⁴ mole par mole du mélange de monomères.

Ces copolymères particuliers sont incorporés dans les compositions de l'invention, de façon préférentielle, sous forme d'émulsions eau dans l'huile contenant de 35 à 40 % en poids de ce copolymère, de 15 à 25 % en poids d'un mélange d'hydrocarbures isoparaffiniques en C₁₂-C₁₃, de 3 à 8 % en poids de lauryléther de polyéthylèneglycol à 7 moles d'oxyde d'éthylène et d'eau. Une telle émulsion est commercialisée sous le nom de "SEPIGEL 305" par la société SEPPIC.

De préférence, les polymères des compositions cosmétiques conformes à la présente invention présentent avantageusement en solution ou en dispersion, à 1 % de matière active dans l'eau, une viscosité mesurée au moyen du rhéomètre Rhéomat RM 180, à 25 °C, supérieure à 0,1 ps, et plus avantageusement encore supérieure à 0,2 cp, à un taux de cisaillement de 200 s⁻¹.

Selon l'invention, le ou les agents épaississants peuvent représenter de 0,001 % à 20 % en poids, de préférence de 0,01 % à 10% en poids et plus particulièrement de 0,1 à 3% en poids par rapport au poids total de la composition finale.

Les compositions de l'invention contiennent en outre avantageusement au moins un agent tensioactif choisi parmi les agents tensioactifs anioniques, amphotères, non-ioniques, ou leurs mélanges qui est généralement présent en une quantité comprise entre 0,1% et 60% en poids environ, de préférence entre 3% et 40% et encore plus préférentiellement entre 5% et 30%, par rapport au poids total de la composition.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.
Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₅) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Coco-amphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

Dans les compositions conformes à l'invention, on utilise de préférence des mélanges d'agents tensioactifs et en particulier des mélanges d'agents tensioactifs anioniques et des mélanges d'agents tensioactifs anioniques et d'agents tensioactifs amphotères ou non ioniques. Un mélange particulièrement préféré est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

Encore plus préférentiellement les compositions selon l'invention peuvent contenir en outre au moins un tensioactif cationique.
Les tensioactifs cationiques peuvent être choisis parmi :
A) les sels d'ammonium quaternaires de la formule générale (IV) suivante : 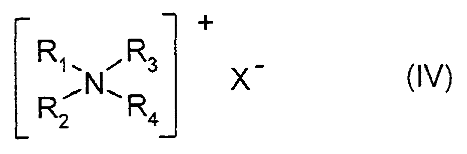 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   i) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   ii) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (V) suivante : 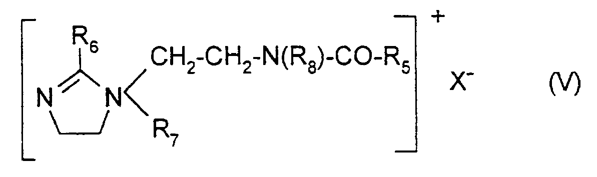 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (VI) : 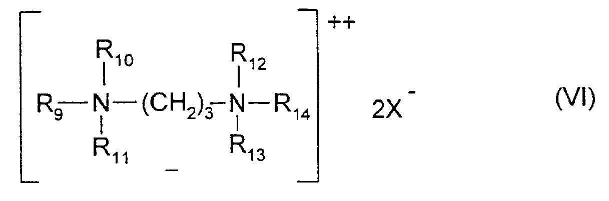 dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (VII ) suivante : 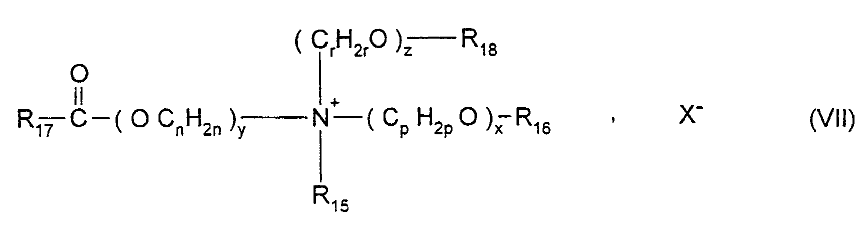 dans laquelle:
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 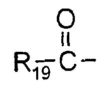
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 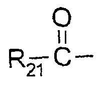
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 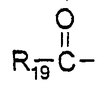
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 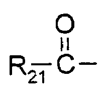
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Le tensioactif cationique est généralement présent dans des concentrations allant de 0,1 à 10% en poids par rapport au poids total de la composition et de préférence de 0,5 à 7 % en poids et plus préférentiellement entre 1 et 5% en poids.

La composition de l'invention peut également contenir au moins un additif choisi parmi les parfums, les agents nacrants, les conservateurs, les filtres solaires siliconés ou non, les vitamines, les provitamines, les polymères cationiques, amphotères, anioniques ou non ioniques, les protéines, les hydrolysats de protéïne, l'acide méthyl-18 eicosanoique, les hydroxyacides, le panthénol, les silicones volatiles ou non volatiles, cycliques ou linéaires ou réticulés, modifiées ou non, les céramides, les pseudocéramides, , les huiles végétales, animales, minérales ou de synthèse et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions d'après-shampooing à rincer ou non.
Les compositions selon l'invention peuvent être également des compositions détergentes telles que des shampooings, des gels-douche, des bains moussants et peuvent être également des démaquillants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères et non ioniques tels que définis ci-dessus.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

Dans les exemples, les noms commerciaux ont les définitions suivantes :

### EXEMPLE 1

On a réalisé un après-shampooing conforme à l'invention de composition suivante :
- Mélange de myristate, palmitate et stéarate de
   myristyle, cétyle et stéaryle 0,5 g
- Chlorure de béhényl triméthyl ammonium
   (GENAMIN KDMP de CLARIANT) 1,1 g MA
- Emulsion cationique à 67% MA de copolymère polydiméthylsiloxane à groupements alpha-oméga vinyle / polydiméthylsiloxane à groupements alpha-oméga hydrogéno (DC-1997 de DOW CORNING) 1,36 gMA
- Mélange d'alcool cétylique et d'alcool stéarylique
   (50/50 en poids) 3g
- Lauryldiméthiconecopolyol à 91% de MA
   (Q2-5200 de DOW CORNING) 0,23 gMA
- Gomme de xanthane 0,2 g
- Parfum,conservateurs qs
- Eau qsp 100 g

On applique cette composition sur des cheveux lavés et essorés. On laisse pauser pendant 2 minutes . On rince abondamment à l'eau.
Les cheveux traités avec cet après-shampooing sont doux, lisses et se démêlent facilement.

## Revendications

1. Composition cosmétique, **caractérisée par le fait qu'**elle comprend, dans un milieu cosmétiquement acceptable, au moins un épaississant non cellulosique choisi parmi les gommes de xanthane, de scléroglucane, de gellane, de rhamsan, les alginates, la maltodextrine, l'amidon et ses dérivés, la gomme de karaya, la farine de caroube, les gommes de guar, les polyéthylèneglycols (PEG) et leurs dérivés, les polyacrylamides,
et au moins une émulsion aqueuse comprenant :
au moins un copolymère siliconé de viscosité comprise entre 10⁶ et 100.10⁶ cP résultant de la réaction d'addition, en présence d'un catalyseur, d'au moins :
- (a) un polysiloxane de formule (I) ; 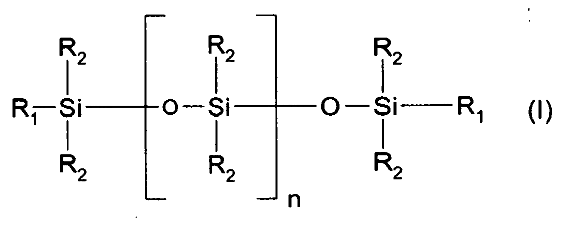 dans laquelle :
R1 désigne un groupement réagissant par réaction d'addition de chaîne tel que par exemple un atome d'hydrogène, un groupement aliphatique ayant une insaturation éthylénique notamment vinyl, allyl ou héxényl,
les groupements R2 de la formule (I) représentent des groupements alkyle ayant de 1 à 20 atomes de carbone, cycloalkyle ayant 5 ou 6 atomes de carbone, aryle, alkylaryle ayant de 7 à 20 atomes de carbone ou hydroxyle, et peuvent comporter en outre, des groupements fonctionnels tels qu'éthers, amines, carboxyles, hydroxyles, thiols, esters, sulfonates, sulfates,
n est un entier tel que le polysiloxane de formule (I) ait de préférence une viscosité cinématique comprise entre 1 et 1.10⁶ mm²/s..
- (b) et d'au moins un composé siliconé comprenant au moins un et au plus deux groupements réagissant avec les groupements R1 du polysiloxane (a),
l'un au moins des composés de type (a) ou (b) contenant un groupe aliphatique ayant une insaturation éthylénique.

2. Composition selon la revendication 1, **caractérisée par le fait que** R2 désigne méthyle.

3. Composition selon l'une des revendications 1 ou 2, **caractérisée par le fait que** le composé de type (b) est un autre polysiloxane de type (a) dans lequel les groupements R1 du polysiloxane (b) réagissent avec les groupement R1 du polysiloxane (a).

4. Composition selon l'une quelconque des revendications 1 à 3, **caractérisée par le fait que** le copolymère siliconé est obtenu par réaction d'addition, en présence d'un catalyseur d'hydrosilylation, d'au moins
- (a) un alpha,oméga-di vinyl polydiméthylsiloxane, et
- (b) d'un alpha,oméga-di hydrogéno polydiméthylsiloxane.

5. Composition selon l'une quelconque des revendications 1 à 4, **caractérisée par le fait que** ladite émulsion du copolymère siliconé a une taille de gouttelettes ou de particules de silicone allant de 10 nm à 50 µm, et de préférence de 0,3 µm à 20 µm.

6. Composition selon l'une quelconque des revendications 1 à 5, **caractérisée par le fait que** l'émulsion aqueuse du copolymère siliconé représente de 0,5 à 15% en poids du poids total de la composition.

7. Composition selon l'une quelconque des revendications 1 à 6, **caractérisée par le fait que** le copolymère siliconé est présent à une concentration comprise entre 0,05 et 10% en poids par rapport au poids total de la composition.

8. Composition selon l'une quelconque des revendications 1 à 7, **caractérisée par le fait que** le polyacrylamide épaississant est choisi parmi :
- les homopolymères de 2-acrylamido-2-méthylpropane sulfonique réticulés,
- les copolymères d'acrylamide et d'acrylate d'ammonium éventuellement réticulés,
- les copolymères d'acrylamide et d'acide 2-acrylamido 2-méthyl propanesulfonique partiellement ou totalement neutralisés éventuellement réticulés.

9. Composition selon l'une quelconque des revendications précédentes, **caractérisée en ce que** l'agent épaississant est présent à une concentration comprise entre 0,001 % et 20 % en poids par rapport au poids total de la composition, de préférence entre 0,01 % et 3 % en poids.

10. Composition selon l'une quelconque des revendications précédentes, **caractérisée en ce que** ledit copolymère siliconé est présent à une concentration comprise entre 0,05 % et 10 % en poids par rapport au poids total de la composition, de préférence entre 0,1 % et 5 % en poids.

11. Composition selon l'une quelconque des revendications précédentes, **caractérisée par le fait qu'**elle comprend en outre au moins un agent tensioactif choisi parmi les tensioactifs anioniques, non ioniques, amphotères et leurs mélanges.

12. Composition selon la revendication 11, **caractérisée par le fait que** le ou les agents tensioactifs sont présents à une concentration comprise entre 0,1% et 60% en poids, de préférence entre 3% et 40% en poids, et encore plus préférentiellement entre 5% et 30% en poids, par rapport au poids total de la composition.

13. Composition selon l'une quelconque des revendications précédentes, **caractérisée par le fait qu'**elle comprend en outre au moins un agent tensioactif cationique.

14. Composition selon la revendication précédente, **caractérisée par le fait que** le tensioactif cationique est présent dans des concentrations allant de 0,1 à 10% en poids par rapport au poids total de la composition et de préférence de 0,5 à 7 % en poids et plus préférentiellement entre 1 et 5% en poids.

15. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

16. Utilisation d'une composition telle que définie dans l'une quelconque des revendications précédentes pour le lavage ou pour le soin des matières kératiniques.

17. Procédé de traitement des matières kératiniques, telles que les cheveux, **caractérisé en ce qu'**il consiste à appliquer sur lesdites matières une composition cosmétique selon l'une des revendications 1 à 15, puis à effectuer éventuellement un rinçage à l'eau.

18. Utilisation d'une émulsion d'au moins un copolymère siliconé tel que défini à l'une des revendications 1 à 5 dans, ou pour la fabrication d'une composition cosmétique comprenant un agent épaississant non cellulosique, tel que défini dans la revendication 1.

## Claims

1. Cosmetic composition, **characterized in that** it comprises, in a cosmetically acceptable medium, at least one non-cellulose-based thickener chosen from xanthan gum, scleroglucan gum, gellan gum, rhamsan gum, alginates, maltodextrin, starch and its derivatives, karaya gum, carob flour, guar gums, polyethylene glycols (PEGs) and their derivatives, and polyacrylamides,
and at least one aqueous emulsion comprising:
at least one silicone copolymer having a viscosity of between 10⁶ and 100 × 10⁶ cP resulting from the addition reaction, in the presence of a catalyst, of at least:
- (a) one polysiloxane of formula (I): 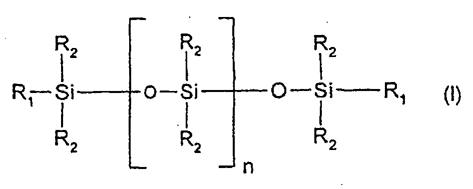 in which:
R1 denotes a group that reacts by chain addition reaction, such as, for example, a hydrogen atom, or an aliphatic group having an ethylenic unsaturation, in particular vinyl, allyl or hexenyl,
the groups R2 of formula (I) represent alkyl groups having from 1 to 20 carbon atoms, cycloalkyl groups having 5 or 6 carbon atoms, aryl groups, alkylaryl groups having from 7 to 20 carbon atoms or hydroxyl groups, and can also comprise functional groups such as ethers, amines, carboxyls, hydroxyls, thiols, esters, sulphonates or sulphates,
n is an integer such that the polysiloxane of formula (I) preferably has a kinematic viscosity of between 1 and 1 × 10⁶ mm²/s,
- (b) and of at least one silicone compound comprising at least one and no more than two groups that react with the groups R1 of the polysiloxane (a),
at least one of the compounds of type (a) or (b) containing an aliphatic group having an ethylenic unsaturation.

2. Composition according to Claim 1, **characterized in that** R2 denotes methyl.

3. Composition according to either of Claims 1 and 2, **characterized in that** the compound of type (b) is another polysiloxane of type (a) in which the groups R1 of the polysiloxane (b) react with the groups R1 of the polysiloxane (a).

4. Composition according to any one of Claims 1 to 3, **characterized in that** the silicone copolymer is obtained by addition reaction, in the presence of a hydrosilylation catalyst, of at least:
- (a) one α,ω-divinylpolydimethylsiloxane, and
- (b) one α,ω-dihydrogenopolydimethylsiloxane.

5. Composition according to any one of Claims 1 to 4, **characterized in that** said emulsion of the silicone copolymer has a silicone droplet or particle size ranging from 10 nm to 50 µm, and preferably from 0.3 µm to 20 µm.

6. Composition according to any one of Claims 1 to 5, **characterized in that** the aqueous emulsion of the silicone copolymer represents from 0.5% to 15% by weight of the total weight of the composition.

7. Composition according to any one of Claims 1 to 6, **characterized in that** the silicone copolymer is present at a concentration of between 0.05% and 10% by weight relative to the total weight of the composition.

8. Composition according to any one of Claims 1 to 7, **characterized in that** the thickening polyacrylamide is chosen from:
- crosslinked 2-acrylamido-2-methylpropane sulphonic homopolymers,
- optionally crosslinked copolymers of acrylamide and of ammonium acrylate,
- optionally crosslinked, partially or totally neutralized copolymers of acrylamide and of 2-acrylamido-2-methylpropanesulphonic acid.

9. Composition according to any one of the preceding claims, **characterized in that** the thickener is present at a concentration of between 0.001% and 20% by weight relative to the total weight of the composition, preferably between 0.01% and 3% by weight.

10. Composition according to any one of the preceding claims, **characterized in that** said silicone copolymer is present at a concentration of between 0.05% and 10% by weight relative to the total weight of the composition, preferably between 0.1% and 5% by weight.

11. Composition according to any one of the preceding claims, **characterized in that** it also comprises at least one surfactant chosen from anionic, nonionic and amphoteric surfactants, and mixtures thereof.

12. Composition according to Claim 11, **characterized in that** the surfactant(s) is (are) present at a concentration of between 0.1% and 60% by weight, preferably between 3% and 40% by weight, and even more preferably between 5% and 30% by weight, relative to the total weight of the composition.

13. Composition according to any one of the preceding claims, **characterized in that** it also comprises at least one cationic surfactant.

14. Composition according to the preceding claim, **characterized in that** the cationic surfactant is present in concentrations ranging from 0.1% to 10% by weight relative to the total weight of the composition, and preferably from 0.5% to 7% by weight, and more preferably between 1% and 5% by weight.

15. Composition according to any one of the preceding claims, **characterized in that** it is in the form of a shampoo, a conditioner, a composition for permanent-waving, straightening, dyeing or bleaching the hair, a rinse-out composition to be applied between the two steps of a permanent-waving or hair-straightening operation, or a washing composition for the body.

16. Use of a composition as defined in any one of the preceding claims, for washing or caring for keratin materials.

17. Process for treating keratin materials, such as the hair, **characterized in that** it consists in applying a cosmetic composition according to one of Claims 1 to 15 to said materials, and then in optionally rinsing it out with water.

18. Use of an emulsion of at least one silicone copolymer as defined in one of Claims 1 to 5, in, or for the manufacture of, a cosmetic composition comprising a non-cellulose-based thickener as defined in Claim 1.

## Patentansprüche

1. Kosmetische Zusammensetzung, **dadurch gekennzeichnet, dass** sie in einem kosmetisch akzeptablen Medium mindestens ein nicht auf Cellulose basierendes Verdickungsmittel, das unter Xanthan, Skleroglucan, Gellan, Rhamsan, Alginaten, Maltodextrin, Stärke und ihren Derivaten, Karaya-Gummi, Johannisbrotkernmehl, Guargummen, Polyethylenglycolen (PEG) und ihren Derivaten und Polyacrylamiden ausgewählt ist, und mindestens eine wässrige Emulsion enthält, die enthält:
mindestens ein Siliconcopolymer mit einer Viskosität von 10⁶ bis 100.10⁶ cP enthält, entstehend in Gegenwart eines Katalysators bei der Addition von zumindest:
(a) einem Polysiloxan der Formel (I): 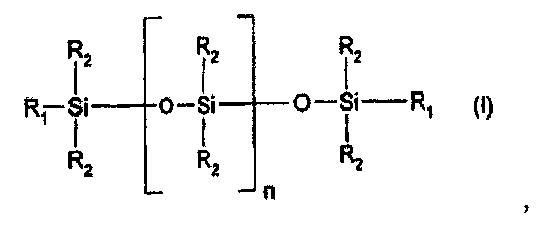 worin bedeuten:
R1 eine Gruppe, die durch Kettenaddition reagiert, wie beispielsweise ein Wasserstoffatom, eine aliphatische ethylenisch ungesättigte Gruppe, besonders Vinyl, Allyl oder Hexenyl,
wobei die Gruppen R2 der Formel (I) Alkylgruppen mit 1 bis 20 Kohlenstoffatomen, Cycloalkylgruppen mit 5 oder 6 Kohlenstoffatomen, Arylgruppen, Alkylarylgruppen mit 7 bis 20 Kohlenstoffatomen oder Hydroxy bedeuten und ferner funktionelle Gruppen enthalten können, wie beispielsweise Ethergruppen, Amingruppen, Carboxygruppen, Hydroxygruppen, Thiolgruppen, Estergruppen, Sulfonatgruppen, Sulfatgruppen,
n eine ganze Zahl, die so gewählt ist, dass das Polysiloxan der Formel (I) vorzugsweise eine kinematische Viskosität von 1 bis 1·10⁶ mm²/s aufweist;
(b) und mindestens einer siliconierten Verbindung, die mindestens eine und höchstens zwei Gruppen aufweist, die mit den Gruppen R1 des Polysiloxans (a) reagieren,
wobei mindestens eine der Verbindungen vom Typ (a) oder (b) eine aliphatische Gruppe enthält, die ethylenisch ungesättigt ist.

2. Zusammensetzung nach Anspruch 1, **dadurch gekennzeichnet, dass** R2 Methyl bedeutet.

3. Zusammensetzung nach einem der Ansprüche 1 oder 2, **dadurch gekennzeichnet, dass** die Verbindung vom Typ (b) ein weiteres Polysiloxan vom Typ (a) ist, wobei die Gruppen R1 des Polysiloxans (b) mit den Gruppen R1 des Polysiloxans (a) reagieren.

4. Zusammensetzung nach einem der Ansprüche 1 bis 3, **dadurch gekennzeichnet, dass** das Siliconcopolymer in Gegenwart eines Hydrosilylierungskatalysators durch Addition hergestellt wird von zumindest:
(a) einem alpha,omega-Divinylpolydimethylsiloxan und
(b) einem alpha,omega-Dihydrogenopolydimethylsiloxan.

5. Zusammensetzung nach einem der Ansprüche 1 bis 4, **dadurch gekennzeichnet, dass** die Emulsion des Siliconcopolymers eine Tröpfchengröße oder Größe der Siliconpartikel im Bereich von 10 nm bis 50 µm und vorzugsweise 0,3 bis 20 µm aufweist.

6. Zusammensetzung nach einem der Ansprüche 1 bis 5, **dadurch gekennzeichnet, dass** die wässrige Emulsion des Siliconcopolymers 0,5 bis 15 Gew.-% des Gesamtgewichts der Zusammensetzung ausmacht.

7. Zusammensetzung nach einem der Ansprüche 1 bis 6, **dadurch gekennzeichnet, dass** das Siliconcopolymer in einer Konzentration von 0,05 bis 10 Gew.-%, bezogen auf das Gesamtgewicht der Zusammensetzung, enthalten ist.

8. Zusammensetzung nach einem der Ansprüche 1 bis 7, **dadurch gekennzeichnet, dass** das verdickende Polyacrylamid ausgewählt ist unter:
- vernetzen Homopolymeren von 2-Acrylamido-2-methylpropansulfonsäure,
- Copolymeren von Acrylamid und Ammoniumacrylat, die gegebenenfalls vernetzt sind,
- Copolymeren von Acrylamid und 2-Acrylamido-2-methylpropansulfonsäure, die ganz oder teilweise neutralisiert und gegebenenfalls vernetzt sind.

9. Zusammensetzung nach einem der vorhergehenden Ansprüche, **dadurch gekennzeichnet, dass** das Verdickungsmittel in einer Konzentration von 0,001 bis 20 Gew.-%, bezogen auf das Gesamtgewicht der Zusammensetzung, und vorzugsweise 0,01 bis 3 Gew.-%, enthalten ist.

10. Zusammensetzung nach einem der vorhergehenden Ansprüche, **dadurch gekennzeichnet, dass** das Siliconcopolymer in einer Konzentration von 0,05 bis 10 Gew.-%, bezogen auf das Gesamtgewicht der Zusammensetzung, und vorzugsweise 0,1 bis 5 Gew.-%, enthalten ist.

11. Zusammensetzung nach einem der vorhergehenden Ansprüche, **dadurch gekennzeichnet, dass** sie ferner mindestens einen grenzflächenaktiven Stoff enthält, der unter den anionischen, nichtionischen oder amphoteren grenzflächenaktiven Stoffen und deren Gemischen ausgewählt ist.

12. Zusammensetzung nach Anspruch 11, **dadurch gekennzeichnet, dass** der oder die grenzflächenaktive(n) Stoff(e) in einer Konzentration von 0,1 bis 60 Gew.-%, vorzugsweise 3 bis 40 Gew.-% und noch bevorzugter 5 bis 30 Gew.-%, bezogen auf das Gesamtgewicht der Zusammensetzung, enthalten sind.

13. Zusammensetzung nach einem der vorhergehenden Ansprüche, **dadurch gekennzeichnet, dass** sie ferner mindestens einen kationischen grenzflächenaktiven Stoff enthält.

14. Zusammensetzung nach dem vorhergehenden Anspruch, **dadurch gekennzeichnet, dass** der kationische grenzflächenaktive Stoff in Konzentrationen von 0,1 bis 10 Gew.-%, bezogen auf das Gesamtgewicht der Zusammensetzung, vorzugsweise 0,5 bis 7 Gew.-% und besonders bevorzugt 1 bis 5 Gew.-% enthalten ist.

15. Zusammensetzung nach einem der vorhergehenden Ansprüche, **dadurch gekennzeichnet, dass** sie als Haarwaschmittel, Konditioner, Zusammensetzung für dauerhafte Verformungen, Entkräuselungen, Färbungen oder Entfärbungen der Haare, Zusammensetzung, die ausgespült wird und zwischen den beiden Schritten einer permanenten Verformung oder Entkräuselung aufgebracht wird oder reinigende Zusammensetzung für den Körper vorliegt.

16. Verwendung einer Zusammensetzung nach einem der vorhergehenden Ansprüche zur Reinigung oder zur Pflege von Keratinsubstanzen.

17. Verfahren zur Behandlung von Keratinsubstanzen, beispielsweise zur Behandlung der Haare, das **dadurch gekennzeichnet ist, dass** es darin besteht, auf diese Substanzen eine kosmetische Zusammensetzung nach einem der Ansprüche 1 bis 15 aufzutragen und dann gegebenenfalls mit Wasser zu spülen.

18. Verwendung einer Emulsion mindestens eines Siliconcopolymers, wie es in einem der Ansprüche 1 bis 5 definiert ist, in einer kosmetischen Zusammensetzung, die ein nicht auf Cellulose basierendes Verdickungsmittel, wie es in Anspruch 1 definiert ist, enthält, oder für die Herstellung einer solchen Zusammensetzung.
